# EUROPÄISCHE PATENTSCHRIFT

(11) **EP 0 516 017 B1**
(45) Veröffentlichungstag und Bekanntmachung des Hinweises auf die Patenterteilung: **18.09.1996**
(21) Anmeldenummer: 92108787.0
(22) Anmeldetag: 25.05.1992
(51) Int. Cl.: C08G 65/28, C08G 65/12, B01J 27/08

(54) **Verfahren zur Herstellung von Alkoxylaten mit enger Homologenverteilung unter Verwendung von Antimonpentahalogenid-Komplexen als Katalysator**
Method for the production of polyalkylene oxides with a narrow molecular weight distribution using an antimony pentahalogen complex as catalyst
Méthode de production de poly(oxyde de alkylène) avec une gamme de poids moléculaire étoite par l'utilisation d'un complexe d'antimoine pentahalogéné comme catalyseur

(30) Priorität: 31.05.1991 DE 4117935
(43) Veröffentlichungstag der Anmeldung: 02.12.1992
(73) Patentinhaber: HOECHST AKTIENGESELLSCHAFT, 65926 Frankfurt am Main (DE)
(72) Erfinder: Wimmer, Ignaz, W-8261 Winhöring (DE); Kupfer, Rainer, Dr., W-8261 Kastl (DE)

(56) Entgegenhaltungen:
- EP-A- 0 172 611
- FR-A- 1 446 042
- US-A- 3 359 331
- US-A- 4 376 063
- DATABASE WPIL Derwent Publications Ltd., London, GB; AN 88-165785(24)

## Beschreibung

Die Erfindung betrifft ein Verfahren zur Herstellung von Alkoxylaten mit enger Homologenverteilung durch Umsetzung von mindestens eine Hydroxylgruppe enthaltenden Verbindungen mit Alkylenoxid in Gegenwart eines Antimonpentahalogenid-Komplexes als Katalysator.

Es ist schon seit langem bekannt, zum Beispiel aus der britischen Patentschrift 796,508, daß Antimonpentahalogenide als Katalysator bei der Umsetzung von aktive H-Atome (zum Beispiel in Form von Hydroxylgruppen) enthaltenden Verbindungen mit Alkylenoxid (zum Beispiel Ethylenoxid und/oder Propylenoxid) zu Alkoxylaten mit enger Homologenverteilung führen. Diesem Vorteil steht als Nachteil nicht nur die mehr oder weniger schwierige Handhabbarkeit von Antimonpentahalogeniden entgegen (stark rauchend, korrosiv, hydrolyseempfindlich und dergleichen), sondern insbesondere die nicht zufriedenstellende Farbqualität des Alkoxylates.

Um die genannten Nachteile von Antimonpentahalogeniden zu überwinden, hat man bereits versucht, Komplexverbindungen von Antimonpentahalogeniden mit Alkoholen, Ethern, Carbonsäuren oder Aminen als Alkoxylierungkatalysator einzusetzen, vergleiche zum Beispiel US-Patentschriften 3,359,331 und 4,375,564. Mit diesen Katalysatoren werden zwar, ähnlich wie mit Antimonpentahalogeniden als solchen, "narrow-range" Alkoxylate erhalten, nachteilig ist aber ihre relativ geringe Aktivität.

Es wurden nun spezielle Antimonpentahalogenid-Komplexe gefunden, die sowohl eine hohe Oxalkylierungsaktivität aufweisen, als auch zu Alkoxylaten mit enger Homologenverteilung und guter Farbqualität führen. Sie sind darüber hinaus leicht und wirtschaftlich herstellbar und problemlos handhabbar. Es handelt sich um Komplexverbindungen aus einem Antimonpentahalogenid und einer bestimmten Lewis Base.

Das erfindungsgemäße Verfahren zur Herstellung von Alkoxylaten mit enger Homologenverteilung durch Umsetzung von mindestens eine Hydroxylgruppe enthaltenden Verbindungen mit Alkylenoxid in Gegenwart eines Antimonpentahalogenid-Komplexes als Katalysator ist dadurch gekennzeichnet, daß ein Komplex aus Antimonpentahalogenid und einer Lewis Base mit einem DS-Wert von 24 bis 36 cm⁻¹, vorzugsweise 24 bis 34 cm⁻¹, eingesetzt wird, wobei der DS-Wert der Lewis Base definiert ist als der Differenzwert zwischen der symmetrischen Streckschwingungsfrequenz des HgBr₂-Moleküls in der Gasphase und in der Lewis Base.

Die erfindungsgemäß einzusetzenden Lewis Basen sind bekannt, vergleiche Arbeit von Ingmar Persson mit dem Titel "On the Coordinating Properties of some Solvents", publiziert in der Zeitschrift 'Inorganica Chimica Acta', 129 (1987), Seiten 183 bis 197. Hier sind die in Rede stehenden DS-Werte ausführlich beschrieben (vergleiche insbesondere Seite 183, linke Spalte, zweiter Absatz, Seite 184, rechte Spalte, vorletzter Absatz und Seite 192, rechte Spalte, letzter Absatz folgend auf Seite 193) und für eine Reihe von entsprechenden Komplexverbindungen im einzelnen angegeben (vergleiche Tabelle V auf Seite 194).

Beim erfindungsgemäßen Verfahren werden also Komplexverbindungen aus einem Antimonpentahalogenid, vorzugsweise Antimonpentabromid oder Antimonpentachlorid, und einer Lewis Base mit einem DS-Wert von 24 bis 36 cm⁻¹, vorzugsweise 24 bis 34 cm⁻¹, eingesetzt. Es ist überraschend, daß gerade diese Lewis Basen die angestrebten vorteilhaften Effekte bringen. Lewis Basen mit einem DS-Wert von weniger als 24 cm⁻¹ und solche mit einem DS-Wert von mehr als 36 cm⁻¹ lassen mehrfach zu wünschen übrig, so zum Beispiel im Hinblick auf die Herstellung der in Rede stehenden Komplexe und/oder im Hinblick auf die katalytische Aktivität der entsprechenden Komplexverbindungen (siehe Vergleichsbeispiele).

Bevorzugte Lewis Basen sind solche, die einen DS-Wert von 24 bis 36 cm⁻¹, vorzugsweise 24 bis 34 cm⁻¹, aufweisen und zur Gruppe der O-Donatoren und N-Donatoren gehören. Als bevorzugte Lewis Basen seien im einzelnen genannt: Di-C₁ bis C₄-alkylsulfoxide, Di-C₁ bis C₄-alkylsulfone, Hexa-C₁ bis C₄-alkylphosphorsäuretriamide, N,N-(di-C₁ bis C₄-alkyl)acylamide und N-C₁ bis C₄-alkylpyrrolidone, wobei "C₁ bis C₄-alkyl" vorzugsweise Methyl oder Ethyl ist und "acyl" vorzugsweise Formyl, Acetyl oder Propionyl ist.

Die Herstellung der erfindungsgemäß einzusetzenden Komplexe aus einem Antimonpentahalogenid, vorzugsweise Antimonpentabromid oder Antimonpentachlorid, und einer der angegebenen Lewis Basen ist einfach. Die exotherme Reaktion zwischen den beiden Komponenten verläuft in kurzer Zeit und quantitativ. In der Regel genügt es, die beiden Komponenten bei einer Temperatur von vorzugsweise 0 bis 50 °C unter Rühren und gegebenenfalls Kühlen (zum Aufrechterhalten der genannten Temperatur) zusammenzubringen, vorzugsweise in Gegenwart eines Lösungsmittels, worauf das ausgefallene Salz, das ist der SbX₅/Lewis-Base-Komplex, abgetrennt (abfiltriert) und getrocknet wird (X = Halogen). Die beiden Komponenten werden vorzugsweise im Molverhältnis von etwa 1 : 1 eingesetzt und das Lösungsmittel in einer 3- bis 15fachen Gewichtsmenge im Vergleich zur Gewichtsmenge an eingesetzter Lewis-Base-Verbindung. Bevorzugte inerte Lösungsmittel sind halogenierte Kohlenwasserstoffe, wie Dichlormethan (Siedepunkt 40 °C), Tetrachlorkohlenstoff (Siedepunkt 77 °C), Dichlorethan (Siedepunkt 84 °C) und/oder Trifluortrichlorethan (Siedepunkt 48 °C). Nach einer bevorzugten Herstellungsweise für die erfindungsgemäß einzusetzenden Komplexe werden die Lewis-Base-Verbindung und das Lösungsmittel in einem Reaktionsgefäß vorgelegt. Zu dieser Mischung wird dann das Antimonpentahalogenid unter Rühren kontinuierlich oder portionsweise dazugegeben unter Aufrechterhalten einer Temperatur von vorzugsweise 0 bis 50 °C und einer Schutzgasatmosphäre, zum Beispiel aus trockenem Stickstoff. Nach der Zugabe des Antimonpentahalogenids liegt der SbX₅/Lewis-Base-Komplex als Salzbrei im eingesetzten Lösungsmittel vor und wird davon abgetrennt, zum Beispiel einfach durch Abfiltrieren oder Abnutschen, und gegebenenfalls noch getrocknet bei einer Temperatur von vorzugsweise 20 bis 80 °C und gegebenenfalls Vakuum zur vollständigen Abtrennung des Lösungsmittels. Die so erhaltene SbX₅/Lewis-Base-Komplexverbindung stellt den erfindungsgemäß einzusetzenden Alkoxylierungskatalysator dar.

Die Menge an erfindungsgemäß einzusetzendem Katalysator kann in weiten Grenzen variieren und liegt im allgemeinen bei 0,0005 bis 0,05 mol pro mol aktive H-Atome enthaltende Verbindung. Mit mehr als 0,05 mol wird in der Regel keine höhere katalytische Wirkung mehr erreicht und mit weniger als 0,0005 mol läßt die katalytische Wirkung bereits beträchtlich nach. Die bevorzugte Menge an SbX₅/Lewis-Base-Komplexkatalysator beträgt demnach 0,001 bis 0,01 mol pro mol aktive H-Atome enthaltende Verbindung.

Die Alkoxylierung von Hydroxylgruppen enthaltenden Verbindungen, das heißt die Umsetzung solcher Verbindungen mit Alkylenoxid, unter Verwendung der beschriebenen Komplexverbindungen als Katalysator wird in üblicher Weise durchgeführt. Man wird also so vorgehen, daß man die zu alkoxylierenden Verbindungen unter Rühren auf eine Temperatur von 40 bis 160 °C bringt, vorzugsweise 60 bis 140 °C, und das Alkylenoxid unter Einhaltung der genannten Temperatur portionsweise oder kontinuierlich zudosiert. Während der Umsetzung kann Normaldruck oder ein erhöhter Druck vorliegen. In der Regel wird die Alkoxylierung bei einem Druck von 2 bis 6 bar durchgeführt. Nach Beendigung der Alkylenoxidzugabe wird die Mischung noch einige Zeit bei der genannten Temperatur zur Nachreaktion gehalten, wobei der Druck immer mehr absinkt und das Ende der Umsetzung anzeigt. Die Menge an Alkylenoxid richtet sich nach dem Zweck, für den das Alkoxylat eingesetzt wird und liegt im allgemeinen bei 1 bis 30 mol, vorzugsweise 2 bis 15 mol und insbesondere 2 bis 8 mol, pro mol zu alkoxylierender Verbindung. Das erhaltene Umsetzungsprodukt kann oft schon als solches, das heißt ohne Abtrennung des Katalysators, eingesetzt werden. Im Falle, daß ein katalysatorfreies Alkoxylat gewonnen werden soll, kann man das Umsetzungsprodukt zum Beispiel einfach mit Wasser oder Lauge zur Zerstörung des Katalysators versetzen und die organische Phase (das ist das Alkoxylat) von der wäßrigen Phase abtrennen. Eine weitere effektive Reinigungsmethode ist in Beispiel 5 beschrieben.

Das erfindungsgemäße Verfahren weist eine Reihe von Vorteilen auf. Aufgrund der hohen katalytischen Aktivität des beschriebenen SbX₅/Lewis-Base-Komplexes wird in relativ kurzer Zeit ein praktisch vollständiger Umsatz erreicht, das heißt eine hohe Ausbeute an Alkoxylat. Das Alkoxylat weist nicht nur eine enge Homologenverteilung auf, sondern auch eine gute Farbqualität. Ein weiterer Vorteil liegt darin, daß die Komplexverbindung problemloser handhabbar und als Katalysator einfacher dosierbar ist als die SbX₅-Verbindung selbst. Die erhaltenen Alkoxylate stellen wertvolle und vielseitig verwendbare Produkte dar, zum Beispiel als Lösungmittel, Tenside (Waschmittel, Reinigungsmittel und dergleichen) und chemische Zwischenprodukte.

Wenn auch die Art der Alkylenoxide und der HO-Gruppen enthaltenden Verbindungen für das erfindungsgemäße Verfahren nicht kritisch sind, sei dazu noch folgendes ausgeführt:

Als Alkylenoxide werden vorzugsweise Ethylenoxid, Propylenoxid und/oder Butylenoxid eingesetzt, wobei Ethylenoxid und/oder Propylenoxid bevorzugt sind. Besonders bevorzugt ist Ethylenoxid.

Als Hydroxylgruppen enthaltende Verbindungen seien Alkohole, Aminoalkohole, Perfluoralkylalkohole, Glykole, Glykolmonoether, Glycerin, Phenole, Kresole und dergleichen genannt, wobei Alkohole bevorzugt sind. Sie können aus nativer Quelle oder aus Synthese-Prozessen stammen, primär, sekundär oder tertiär, linear oder verzweigt, gesättigt oder ungesättigt, ein- oder mehrwertig sein, zum Beispiel Oxo-Alkohole, Guerbet-Alkohole, Ziegler-Alkohole, Fettalkohole und dergleichen. Bevorzugte Alkohole sind die primären oder sekundären, geradkettigen oder verzweigten C₃ bis C₂₄-Alkanole, vorzugsweise C₆ bis C₁₈-Alkanole. Als Beispiele für die bevorzugten Alkohole seien genannt: Butanol, Amylalkohol, Hexanol, Nonanol, Isononylalkohol, Decanol, Undecanol, Isoundecanol, Laurylalkohol, Isotridecylalkohol, Stearylalkohol, Kokosfettalkohol und Gemische davon, ferner 2-Ethylhexanol, 2-Hexyldecanol, 2-Octyldecanol und ähnliche Guerbet-Alkohole. Wie sich herausgestellt hat, werden mit dem beschriebenen Komplexkatalysator auch im Falle von ungesättigten Alkoholen und im Falle von Perfluoralkoholen Alkoxylate mit hoher Ausbeute, enger Homologenverteilung und guter Farbe erhalten. Dies ist um so mehr überraschend, als gerade in diesen Fällen Antimonpentahalogenide als Katalysator mehr oder weniger versagen. Bevorzugte ungesättigte Alkohole sind die primären, geradkettigen oder verzweigten C₃ bis C₂₄-Alkohole, vorzugsweise C₆ bis C₁₈-Alkohole, mit 1 bis 3 Doppelbindungen und bevorzugte Perfluoralkohole sind solche der Formel R_{f}(CH₂)ₓ-OH, worin R_{f} C₄F₉ bis C₁₈F₃₇, vorzugsweise C₆F₁₃ bis C₁₆F₃₃, und x 1, 2, 3 oder 4, vorzugsweise 2, ist.

Die Erfindung wird nun an Beispielen und Vergleichsbeispielen noch näher erläutert, wobei zuerst die Herstellung von erfindungsgemäß einzusetzenden Antimonpentahalogenid/Lewis-Basen-Komplexen beschrieben wird und dann die Alkoxylierung von aktive H-Atome enthaltenden Verbindungen unter Verwendung dieser Komplexe als Katalysator. Die Vergleichsbeispiele sollen den Auswahlcharakter der erfindungsgemäß einzusetzenden Komplexverbindungen untermauern.

### Beispiel 1

### Herstellung eines SbCl₅/Dimethylsulfoxid-Komplexes (Katalysator A):

Zu einer Mischung von 7,65 g (0,098 mol) Dimethylsulfoxid in 50 g Dichlormethan wurden bei einer Temperatur von 30 bis 50 °C 29,20 g (0,098 mol) SbCl₅ unter Rühren und unter trockenem Stickstoff als Schutzgas kontinuierlich zugetropft. Der gebildete Salzbrei wurde von der überstehenden Dichlormethan-Schicht abfiltriert und getrocknet. Die erhaltene SbCl₅/Dimethylsulfoxid-Komplexverbindung = Katalysator A hat die theoretische Molmasse 377.

### Beispiel 2

### Herstellung eines SbCl₅/Hexamethylphosphorsäuretriamid-Komplexes (Katalysator B):

Zu einer Mischung von 17,90 g (0,10 mol) Hexamethylphosphorsäuretriamid in 100 g Dichlormethan wurden bei einer Temperatur von 20 bis 40 °C 29,90 g (0,10 mol) SbCl₅ unter Rühren und unter trockenem Stickstoff als Schutzgas kontinuierlich zugetropft. Der gebildete Salzbrei wurde von der überstehenden Dichlormethan-Schicht abfiltriert und getrocknet. Die erhaltene SbCl₅/Hexamethylphosphorsäuretriamid-Komplexverbindung = Katalysator B hat die theoretische Molmasse von 478.

### Beispiel 3

### Herstellung eines SbCl₅/N,N-Dimethylacetamid-Komplexes (Katalysator C):

Zu einer Mischung von 8,70 g (0,10 mol) N,N-Dimethylacetamid in 50 g Dichlormethan wurden bei einer Temperatur von etwa 0 °C 29,90 g (0,10 mol) SbCl₅, gelöst in 50 g Dichlormethan, unter Rühren und trockenem Stickstoff als Schutzgas kontinuierlich zugetropft. Nach der Zugabe der SbCl₅-Lösung wurde die Mischung mit 50 g Diethylether versetzt, um den gebildeten Komplex auszufällen. Der Salzbrei wurde von der überstehenden Lösungsmittelschicht abfiltriert und getrocknet. Die erhaltene SbCl₅/Dimethylacetamid-Komplexverbindung = Katalysator C hat die theoretische Molmasse von 386.

### Beispiel 4

### Herstellung eines SbCl₅/N-Methyl(2)pyrrolidon-Komplexes (Katalysator D):

Die Herstellung erfolgte wie im Beispiel 2, wobei 9,92 g (0,10 mol) N-Methylpyrrolidon und 29,90 g (0,10 mol) SbCl₅ eingesetzt wurden. Die erhaltene SbCl₅/N-Methylpyrrolidon-Komplexverbindung = Katalysator D hat die theoretische Molmasse von 398.

### Beispiel 5

In einem 1-Liter-Rührautoklaven wurden 198,0 g (1,0 mol) von einem n-C₁₂ bis C₁₄-Alkanol-Gemisch und 1,13 g (0,003 mol) vom Katalysator A vorgelegt. In die auf 80 °C erhitzte Mischung wurden bei einer Temperatur von anfangs 80 °C und bis auf 120 °C ansteigend 176,0 g (4,0 mol) Ethylenoxid in dem Maße zudosiert, wie es abreagierte (der Druck im Autoklaven lag bei 2 bis 3 bar), wozu eine Zeit von nur 3 Stunden erforderlich war. Nach der Zugabe des Ethylenoxids wurde die Mischung noch 2 Stunden bei 120 °C zur Nachreaktion gerührt, worauf der Druck bei 1 bar konstant blieb und das Ende der Reaktion anzeigte. Der Autoklaveninhalt (das Reaktionsprodukt) wurde nun zur Zerstörung des Katalysators mit 12 g 10%iger NaOH-Lösung versetzt, bei 130 °C unter Eigendruck 2 Stunden gerührt, danach mit Phosphorsäure auf pH 6,6 eingestellt und das Wasser unter Vakuum abgezogen (getrocknet). Nach Filtration wurden 370 g Ethoxylat erhalten. Das Ethoxylat hatte eine Hazen-Farbzahl von 60 (gemessen nach DIN 53 409) und der Gehalt an freiem Alkanol war 0,6 Gew.-% (die Bestimmung des Alkanolgehaltes erfolgte gaschromatographisch).

### Beispiel 6

In einem 1-Liter-Rührautoklaven wurden 174,0 g (1,0 mol) von einem n-C₁₁ und iso-C₁₁-Alkanol-Gemisch und 1,91 g (0,004 mol) vom Katalysator B vorgelegt. In die auf 80 °C erhitzte Mischung wurden bei einer Temperatur von anfangs 80 °C und bis auf 120 °C ansteigend 352,0 g (8,0 mol) Ethylenoxid in dem Maße zudosiert, wie es abreagierte, wozu eine Zeit von nur 4 Stunden erforderlich war. Die weitere Behandlung und Aufarbeitung erfolgte wie in Beispiel 5. Es wurden 523 g Ethoxylat erhalten. Es hatte eine Hazen-Farbzahl von 60 und der Gehalt an freiem Alkanol war 0,1 Gew.-%.

### Beispiel 7

In einem 1-Liter-Rührautoklaven wurden 529,0 g (2,0 mol) von einem ungesättigten n-C₁₆ bis C₁₈-Alkohol-Gemisch (Iodzahl 75) und 3,90 g (0,01 mol) vom Katalysator C vorgelegt. In die auf 70 °C erhitzte Mischung wurden bei dieser Temperatur 264,0 g (6,0 mol) Ethylenoxid in dem Maße zudosiert, wie es abreagierte, wozu eine Zeit von nur 3 Stunden erforderlich war. Die weitere Behandlung und Aufarbeitung erfolgte wie in Beispiel 5. Es wurden 785 g Ethoxylat erhalten. Es hatte eine Hazen-Farbzahl von 160 und der Gehalt an freiem Alkanol war 4 Gew.-%.

### Beispiel 8

In einem 1-Liter-Rührautoklaven wurden 464,0 g (1,0 mol) Perfluoroctylethanol (C₈F₁₇C₂H₄OH) und 2,0 g (0,005 mol) vom Katalysator D vorgelegt. In die auf 80 °C erhitzte Mischung wurden bei einer Temperatur von 80 bis 90 °C 176,0 g (4,0 mol) Ethylenoxid in dem Maße zudosiert, wie es abreagierte, wozu eine Zeit von nur 3 Stunden erforderlich war. Die weitere Behandlung und Aufarbeitung erfolgte wie in Beispiel 5. Es wurden 641 g Perfluoroctylethanol-Ethoxylat erhalten. Es hatte eine Hazen-Farbzahl von 60 und der Gehalt an freiem Alkohol war 2,2 Gew.-%.

### Beispiel 9

In einem 1-Liter-Rührautoklaven wurden 260,0 g (2,0 mol) von einem 2-Ethylhexanol und 1,60 g (0,004 mol) vom Katalysator D vorgelegt. In die auf 60 °C erhitzte Mischung wurden bei einer Temperatur von 60 bis 80 °C 176,0 g (4,0 mol) Ethylenoxid in dem Maße zudosiert, wie es abreagierte, wozu eine Zeit von nur 2 Stunden erforderlich war. Die weitere Behandlung und Aufarbeitung erfolgte wie in Beispiel 5. Es wurden 430 g Ethoxylat erhalten. Es hatte eine Hazen-Farbzahl von 100 und der Gehalt an freiem 2-Ethylhexanol war 4,1 Gew.-%.

Die Ethoxylate der Beispiele 5 bis 9 weisen, wie gaschromatographisch festgestellt wurde, die angestrebte enge Homologenverteilung auf.

### Vergleichsbeispiel 1

Beispiel 5 wurde wiederholt, mit dem Unterschied, daß anstelle des Katalysators A 1,12 g (0,003 mol) SbCl₅-Diethylether-Komplex als Katalysator eingesetzt wurden (der DS-Wert von Diethylether ist 12 cm⁻¹). Die Zudosierzeit betrug nicht 3, sondern 5 Stunden. Das Ethoxylat hatte eine Hazen-Farbzahl von 140 und der Gehalt an freiem Alkohol betrug 0,7 Gew.-%.

### Vergleichsbeispiel 2

Es wurde wie in Beispiel 7 gearbeitet, mit dem Unterschied, daß anstelle des Katalysators C 3,0 g (0,01 mol) SbCl₅ pur eingesetzt wurden. Beim Zudosieren der 264,0 g (6,0 mol) Ethylenoxid in die auf 70 °C erwärmte Mischung mit dem ungesättigten C₁₆ bis C₁₈-Alkohol zeigte sich, daß nach Zufuhr von etwa 44 g (0,5 mol) Ethylenoxid die Reaktion zum Stillstand kam.

### Vergleichsbeispiel 3

Es wurde wie in Beispiel 8 gearbeitet, mit dem Unterschied, daß anstelle des Katalysators D 1,50 g (0,005 mol) SbCl₅ pur eingesetzt wurden. Der Gehalt an freiem Alkohol im erhaltenen Perfluoroctylethanolethoxylat war nicht 2,2 Gew.-% wie in Beispiel 8, sondern 7,4 Gew.-%

### Vergleichsbeispiel 4

Beispiel 5 wurde wiederholt, mit dem Unterschied, daß anstelle des Katalysators A 1,17 g (0,003 mol) SbCl₅-Pyridin-Komplex als Katalysator eingesetzt wurden (der DS-Wert von Pyridin ist 38 cm⁻¹). Beim Zudosieren des Ethylenoxids in die auf 80 °C erwärmte Mischung mit dem C₁₂ bis C₁₄-Alkohol wurde die Reaktion nach Zufuhr von etwa 44 g (0,5 mol) Ethylenoxid sehr langsam und kam dann zum Stillstand.

### Vergleichsbeispiel 5

Beispiel 5 wurde wiederholt, mit dem Unterschied, daß anstelle des Katalysators A 1,20 g (0,003 mol) SbCl₅-Triethylamin-Komplex als Katalysator eingesetzt wurden (der DS-Wert von Triethylamin ist 23 cm⁻¹). Beim Zudosieren des Ethylenoxids in die auf 80 °C erwärmte Mischung mit dem C₁₂ bis C₁₄-Alkohol kam die Reaktion nach Zufuhr von etwa 44 g (0,5 mol) Ethylenoxid zum Stillstand.

## Patentansprüche

1. Verfahren zur Herstellung von Alkoxylaten mit enger Homologenverteilung durch Umsetzung von mindestens eine Hydroxylgruppe enthaltenden Verbindungen mit Alkylenoxid in Gegenwart eines Antimonpentahalogenid-Komplexes als Katalysator, dadurch gekennzeichnet, daß ein Komplex aus Antimonpentahalogenid und einer Lewis Base mit einem DS-Wert von 24 bis 36 cm⁻¹ eingesetzt wird, wobei der DS-Wert der Lewis Base definiert ist als der Differenzwert zwischen der symmetrischen Streckschwingungsfreguenz des HgBr₂-Moleküls in der Gasphase und in der Lewis Base.

2. Verfahren nach Anspruch 1, dadurch gekennzeichnet, daß ein Komplex aus Antimonpentahalogenid und einer Lewis Base mit einem DS-Wert von 24 bis 34 cm⁻¹ eingesetzt wird.

3. Verfahren nach Anspruch 1 oder 2, dadurch gekennzeichnet, daß ein Komplex aus Antimonpentahalogenid und einer Lewis Base eingesetzt wird, wobei die Lewis Base ausgewählt ist aus der Gruppe der O-Donatoren und N-Donatoren.

4. Verfahren nach Anspruch 1, dadurch gekennzeichnet, daß ein Komplex aus Antimonpentahalogenid und einer Lewis Base eingesetzt wird, wobei die Lewis Base ausgewählt ist aus der Gruppe bestehend aus
Di-C₁ bis C₄-alkylsulfoxiden,
Di-C₁ bis C₄-alkylsulfonen,
Hexa-C₁ bis C₄-alkylphosphorsäuretriamiden,
N,N-(di-C₁ bis C₄-alkyl)acylamiden und
N-C₁ bis C₄-alkylpyrrolidonen.

5. Verfahren nach einem oder mehreren der Ansprüche 1 bis 4, dadurch gekennzeichnet, daß der Katalysator in einer Menge von 0,0005 bis 0,05 mol pro mol Hydroxylgruppen enthaltende Verbindungen eingesetzt wird.

6. Verfahren nach einem oder mehreren der Ansprüche 1 bis 5, dadurch gekennzeichnet, daß als Antimonpentahalogenid Antimonpentabromid oder Antimonpentachlorid, als Alkylenoxid Ethylenoxid oder Propylenoxid und als Hydroxylgruppen enthaltende Verbindungen geradkettige oder verzweigte, gesättigte oder ungesättigte primäre C₃ bis C₂₄-Alkanole oder Perfluoralkylalkohole der Formel R_{f}(CH₂)ₓ-OH eingesetzt werden, worin R_{f} C₄F₉ bis C₁₈F₃₇ und x eine ganze Zahl von 1 bis 4 ist.

7. Verfahren nach einem oder mehreren der Ansprüche 1 bis 6, dadurch gekennzeichnet, daß die Alkoxylierung bei einer Temperatur von 40 bis 160 °C und einem Druck von 2 bis 6 bar durchgeführt wird.

## Claims

1. A process for the preparation of alkoxylates having a narrow distribution of homologs by reaction of compounds containing at least one hydroxyl group with alkylene oxide in the presence of an antimony pentahalide complex as the catalyst, which comprises using a complex of antimony pentahalide with a Lewis base having a DS value of 24 to 36 cm⁻¹, in which the DS value of the Lewis base is defined as the difference in value between the symmetrical stretching frequency of the HgBr₂ molecule in the gas phase and in the Lewis base.

2. The process as claimed in claim 1, wherein a complex of antimony pentahalide with a Lewis base having a DS value of 24 to 34 cm⁻¹ is used.

3. The process as claimed in claim 1 or 2, wherein a complex of antimony pentahalide with a Lewis base is used, in which the Lewis base is selected from the group comprising O donors and N donors.

4. The process as claimed in claim 1, wherein a complex of antimony pentahalide with a Lewis base is used, in which the Lewis base is selected from the group comprising di-C₁- to C₄-alkyl sulfoxides, di-C₁- to C₄-alkyl sulfones, hexa-C₁- to C₄-alkylphosphoric triamides, N,N-(di-C₁- to C₄-alkyl)acylamides and N-C₁- to C₄-alkylpyrrolidones.

5. The process as claimed in one or more of claims 1 to 4, wherein the catalyst is used in an amount of 0.0005 to 0.05 mol per mole of compounds containing hydroxyl groups.

6. The process as claimed in one or more of claims 1 to 5, wherein the antimony pentahalide used is antimony pentabromide or antimony pentachloride, the alkylene oxide used is ethylene oxide or propylene oxide and the compounds containing hydroxyl groups used are straight-chain or branched, saturated or unsaturated primary C₃- to C₂₄-alkanols or perfluoroalkyl alcohols of the formula R_{f}(CH₂)ₓ-OH, in which R_{f} is C₄F₉ to C₁₈F₃₇ and x is an integer from 1 to 4.

7. The process as claimed in one or more of claims 1 to 6, wherein the alkoxylation is carried out at a temperature of 40 to 160°C and a pressure of 2 to 6 bar.

## Revendications

1. Procédé pour la préparation d'alcoxylates ayant une distribution homologue étroite par réaction d'un composé contenant au moins un groupe hydroxyle avec un oxyde d'alkylène en présence d'un complexe de pentahalogénure d'antimoine en tant que catalyseur, caractérisé en ce que l'on utilise un complexe de pentahalogénure d'antimoine et d'une base de Lewis ayant une valeur de déformation vibrationnelle (DS) de 24 à 36 cm⁻¹, en définissant la valeur DS de la base de Lewis comme valeur de la différence entre la fréquence de vibrations d'allongement symétrique de la molécule HgBr₂ en phase gazeuse et de la base de Lewis.

2. Procédé selon la revendication 1, caractérisé en ce que l'on utilise un complexe de pentahalogénure d'antimoine et d'une base de Lewis ayant une valeur DS de 24 à 34 cm⁻¹.

3. Procédé selon la revendication 1 ou 2, caractérisé en ce que l'on utilise un complexe de pentahalogénure d'antimoine et d'une base de Lewis, la base de Lewis étant prise dans un groupe de donneurs de O et de donneurs de N.

4. Procédé selon la revendication 1, caractérisé en ce que l'on utilise un complexe de pentahalogénure d'antimoine et d'une base de Lewis en prenant la base de Lewis du groupe composé de
Di-(alkyl en C₁ à C₄)sulfoxydes,
Di-(alkyl en C₁ à C₄)sulfonates,
Hexa-(alkyl en C₁ à C₄)phosphortriamides,
N,N-di-(alkyl en C₁ à C₄)-acylamides et
N-(alkyl en C₁ à C₄)pyrrolidones.

5. Procédé selon une ou plusieurs des revendications 1 à 4, caractérisé en ce que l'on utilise le catalyseur dans une quantité de 0,0005 à 0,05 mole par mole de composés contenant des groupes hydroxyles.

6. Procédé selon une ou plusieurs des revendications 1 à 5, caractérisé en ce que l'on utilise en tant que pentahalogénure d'antimoine le pentabromure d'antimoine ou le pentachlorure d'antimoine, en tant qu'oxyde d'alkylène l'oxyde d'éthylène ou l'oxyde de propylène et en tant que composés contenant des groupes hydroxyles des alcanols en C₃-C₂₄ primaire, linéaires ou ramifiés, saturés ou insaturés, ou des perfluoroalkylalcools de formule R_{f}(CH₂)ₓ-OH, dans laquelle R_{f} représente C₄F₉ à C₁₈F₃₇ et x vaut de 1 à 4.

7. Procédé selon l'une ou plusieurs des revendications 1 à 6, caractérisé en ce que l'on met en oeuvre l'alcoxylation à une température de 40 à 160 °C et une pression de 2 à 6 bars.
